(19) **Europäisches Patentamt / European Patent Office / Office européen des brevets**

(11) **EP 1 628 614 B1**

(12) **EUROPEAN PATENT SPECIFICATION**

(45) Date of publication and mention of the grant of the patent:
**10.08.2011 Bulletin 2011/32**

(51) Int Cl.:
**A61F 13/56** (2006.01)    **A61F 15/00** (2006.01)

(21) Application number: **04753497.9**

(86) International application number:
**PCT/US2004/016678**

(22) Date of filing: **27.05.2004**

(87) International publication number:
**WO 2004/108043 (16.12.2004 Gazette 2004/51)**

(54) **DISPOSABLE ABSORBENT ARTICLES CONTAINED IN PACKAGE HAVING TRANSPARENT WINDOW**

SAUGFÄHIGE EINWEGARTIKEL IN EINER VERPACKUNG MIT TRANSPARENTEM FENSTER

ARTICLES ABSORBANTS JETABLES CONTENUS DANS UN EMBALLAGE PRESENTANT UNE FENETRE TRANSPARENTE

(84) Designated Contracting States:
**AT BE BG CH CY CZ DE DK EE ES FI FR GB GR HU IE IT LI LU MC NL PL PT RO SE SI SK TR**

(30) Priority: **30.05.2003 US 474489 P**

(43) Date of publication of application:
**01.03.2006 Bulletin 2006/09**

(73) Proprietor: **THE PROCTER & GAMBLE COMPANY Cincinnati, OH 45202 (US)**

(72) Inventors:
• **OHI, Kenji**
  **Kobe,**
  **Hyogo 658-0046 (JP)**
• **MIURA, Tsunetoshi**
  **Kobe,**
  **Hyogo 650-0022 (JP)**

• **ITO, Kensuke**
  **Kobe,**
  **Hyogo 651-1147 (JP)**
• **SASAKI, Akiko**
  **Nishinomiya, Hyogo 663-8153 (JP)**
• **NUMAGA, Mina**
  **Kobe,**
  **Hyogo 655-0046 (JP)**

(74) Representative: **Kremer, Véronique Marie Joséphine et al**
**Procter & Gamble Service GmbH**
**Sulzbacher Strasse 40-50**
**65824 Schwalbach am Taunus (DE)**

(56) References cited:
**WO-A-02/096331        WO-A-03/026543**
**GB-A- 1 100 033        US-A- 3 803 332**
**US-A1- 2002 148 749**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 628 614 B1

## Description

### FIELD OF THE INVENTION

The present invention relates to disposable absorbent articles. More particularly, the present invention relates to a disposable absorbent product containing disposable absorbent articles in a package having a transparent window.

### BACKGROUND OF THE INVENTION

Disposable absorbent articles such as sanitary napkins, pantiliners, diapers and incontinent pads are devices that are typically worn in the crotch region of an undergarment. More specifically, sanitary napkins and pantiliners, for example, are worn by women in a pair of panties that is normally positioned between the wearer's legs, adjacent to the perineum area. They are designed to absorb and retain body fluids or discharges (e.g., urine and menses) from the body of women and to prevent body and clothing from soiling. A wide variety of disposable absorbent articles is currently used by many users for the collection of body fluids.

It is known in the art that sanitary napkins and pantiliners are often individually wrapped by a wrapper sheet to form an individually wrapped configuration. Such individually wrapped sanitary napkins and pantiliners are often preferred by consumers since they can provide cleanness (including their clean image on appearance) of the products. Such individual wrapping technologies or structures are disclosed, for example, USP. Nos. 6,074,376 issued to Mills on June 13, 2000, 5,569,228 issued to Byrd et al. on October 29, 1996 and 5,413,568 issued to Roach et al. on May 9, 1995. After each disposable absorbent article is individually wrapped by a wrapper sheet, the individually wrapped disposable absorbent articles are typically stacked and contained in a package such as a flexible bag or a cardboard box. These packages are convenient to handle the absorbent articles contained at not only the markets but also users' homes.

Recently, it has been recognized that packages having a transparent window(s) are preferred by consumers (or users) since such a transparent window(s) can actually show the absorbent articles contained in the package and thus consumers can directly get the image and/or information about the absorbent articles (e.g., the sizes and the thickness of the absorbent articles) through the window. This is particularly important for recent disposable absorbent articles which have aesthetic feature(s) (e.g., a printed graphic(s) thereon) to get consumers' strong attention.

WO02/096331 discloses disposable absorbent articles contained in a package having window.

However, it has also been found that providing a transparent window(s) in packages may cause serious problems to the absorbent articles in the package. Specifically, the disposable absorbent articles in the package tend to be affected by ultraviolet light which comes into the package through the transparent window(s). This problem becomes particularly significant if the wrapper sheet material of the individually wrapped absorbent articles has low level of protection against the ultraviolet light (i.e., high light transmittance). For example, if the wrapper sheet is formed by a nonwoven material (which generally has low level of protection against the ultraviolet light), component materials of the disposable absorbent articles such as adhesive materials tend to be easily affected by the ultraviolet light since the nonwoven material does not provide enough protection against the ultraviolet light. This typically causes an yellowing (i.e., a color change) or sometimes stiffening of the adhesive materials, and thus damaging the good appearance of the contained absorbent articles as well as their quality.

Thus, there is a need for a disposable absorbent product containing absorbent articles in a package that has enough protection against ultraviolet light. There is also another need for a disposable absorbent product containing absorbent articles in a package that can prevent yellowing or stiffening of adhesive materials used in the absorbent articles.

### SUMMARY OF THE INVENTION

The invention is directed to a disposable absorbent product, comprising: a package having a transparent window; and a plurality of individually wrapped disposable absorbent articles which are stacked and contained in the package. Each absorbent article has a longitudinal centerline and a transverse centerline, and includes (1) a liquid impermeable backsheet having and a garment facing surface, (2) an adhesive means disposed in the longitudinal direction, and (3) a wrapper sheet for individually wrapping the absorbent article. The adhesive means is disposed between the garment facing surface of the backsheet and the wrapper sheet. The adhesive means includes a plurality of adhesive sections which are spaced apart one another by at least one non-adhesive section. The wrapper sheet covers the adhesive means and the garment facing surface of the backsheet. Each absorbent article is folded about at least one folding line that intersects the adhesive means at the non-adhesive section. The stacked plurality of the individually wrapped absorbent articles are arranged associated with the transparent window such that the folding line of each absorbent article positions towards the transparent window of the package.

Since each absorbent article is folded about the fold line that intersects the adhesive means at the non-adhesive section, the individually wrapped and folded absorbent article has at least one edge portion that contains no adhesive section. In the package, the edge portion that contains no adhesive section positions towards the transparent window of the package. As result, the adhesive means can be protected from yellowing caused by the ultraviolet light which comes in through the transparent window.

BRIEF DESCRIPTION OF THE DRAWINGS

Fig. 1 is a top plan view of a sanitary napkin which is one preferred embodiment of the present invention having a portion cut away to reveal an underlying structure;
Fig. 2 is a bottom plan view of the sanitary napkin of Fig. 1 (wherein the removable release liner and wrapper sheet are removed for better understanding);
Fig. 3 is a bottom plan view of the sanitary napkin of Fig. 1 (wherein the wrapper sheet is removed for better understanding);
Fig. 4 is a bottom plan view of the sanitary napkin of Fig. 1 (with the wrapper sheet);
Fig. 5 is a perspective view of the sanitary napkin shown in Fig. 1 which is wrapped by a wrapper sheet;
Fig. 6 is a bottom plan view of a sanitary napkin (wherein the wrapper sheet is removed for better understanding) which is another preferred embodiment of the present invention;
Fig. 7 is a bottom plan view of the sanitary napkin of Fig. 6 (with the wrapper sheet); and
Fig. 8 is a perspective view of a disposable absorbent product containing stacked sanitary napkins which is one preferred embodiment of the present invention.

DETAILED DESCRIPTION OF THE INVENTION

Herein, "comprise" and "include" mean that other elements and/or other steps which do not affect the end result can be added. Each of these terms encompasses the terms "consisting of" and "consisting essentially of".
Herein, "absorbent article" refers to articles which absorb and contain body exudates or discharges such as body fluids, and is intended to include sanitary napkins, pantiliners, diapers, and incontinence pads (and other articles worn in the crotch region of a garment). The invention is preferably applied to a sanitary napkin or a pantyliner.
Herein, "disposable" refers to articles which are intended to be discarded after a single use, composted, or otherwise disposed of in an environmentally compatible manner. (That is, they are not intended to be laundered or otherwise restored or reused as an absorbent article.)
Herein, "sanitary napkin" refers to articles which are worn by females adjacent to the pudendal region which are intended to absorb and contain the various exudates which are discharged from the body (e.g., blood, menses, and urine).
Herein, "joined" encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element.
Herein, "body facing surface" refers to surfaces of absorbent articles and/or their component members which face the body of the wearer, while the term "garment facing surface" refers to the opposite surfaces of the absorbent articles and/or their component members that face away from the wearer when the absorbent articles are worn. Absorbent articles and components thereof, including the topsheet, backsheet, absorbent core, and any individual layers of their components, have a body facing surface and a garment facing surface.

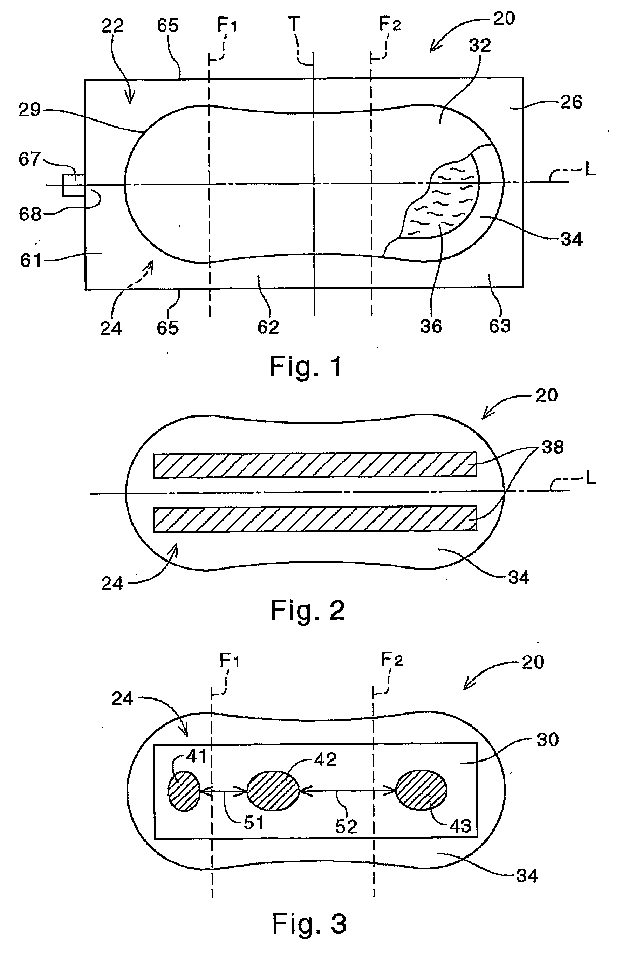

Fig. 1 is a top plan view of a sanitary napkin 20 (i.e., a disposable absorbent article) which is one preferred embodiment of the present invention. Referring to Fig. 1, the sanitary napkin 20 (and its component members) has a body facing surface 22, and a garment facing surface 24 opposed to the body facing surface 22. The sanitary napkin 20 includes three primary components, i.e., a liquid permeable topsheet 32, a liquid impermeable backsheet 34, and an absorbent core 36 disposed between the topsheet 32 and the backsheet 34. The portion formed by these three primary components is hereinafter referred to as "the main body portion" of the sanitary napkin 20.
The sanitary napkin 20 has two centerlines, a longitudinal centerline L and a transverse centerline T. Herein, "longitudinal" refers to a line, axis or direction in the plane of the sanitary napkin 20 that is generally aligned with (e.g., approximately parallel to) a vertical plane which bisects a standing wearer into left and right body halves when the sanitary napkin 20 is worn. Herein, "transverse" or "lateral", are interchangeable, and refer to a line, axis or direction which lies within the plane of the sanitary napkin 20 that is generally perpendicular to the longitudinal direction.
The main body portion of the sanitary napkin 20 is placed on the body facing surface 22 of the wrapper sheet 26. The sanitary napkin 20 has a circumferential edge 29 which defines the outermost edge of the main body portion of the sanitary napkin 20.

The sanitary napkin 20 further includes an adhesive means (not shown in Fig. 1) disposed in the longitudinal direction, and (2) a wrapper sheet 26 for individually wrapping the sanitary napkin 20. It should be noted that the adhesive means can include any type of adhesive material for any purpose which is disposed between the garment facing surface 24 of the backsheet 34 and the wrapper sheet 26.

In the preferred embodiments discussed below, the adhesive means typically includes an adhesive securing means and/or an adhesive fastener, each (or both) of which includes a plurality of adhesive sections which are spaced apart one another by at least one non-adhesive section, as discussed in detail hereinafter. The wrapper sheet 26 covers the adhesive means and the garment facing surface 24 of the sanitary napkin 20. The sanitary napkin 20 will be folded along at least one folding line that intersects the adhesive means at the non-adhesive section.

Fig. 2 is a bottom plan view of the sanitary napkin 20 of Fig. 1 wherein the adhesive fastener and wrapper sheet are removed for better understanding. The sanitary napkin 20 further includes an adhesive fastener 38 disposed on the garment facing surface 24 of the backsheet 34 for attaching the sanitary napkin 20 to the wearer's undergarment. Such an adhesive fastener 38 is often referred to as a central pad adhesive or a panty fastening adhesive. Any adhesive materials known in the art can be used for this adhesive fastener 38. Preferably, pressure sensitive adhesives are used for the adhesive fastener 38. A particularly preferred material for the adhesive fastener is a pressure sensitive adhesive supplied from H. B. Fuller Japan Co., Ltd., Hamamatsu, Japan, under Code No. HL-1461-AZP.

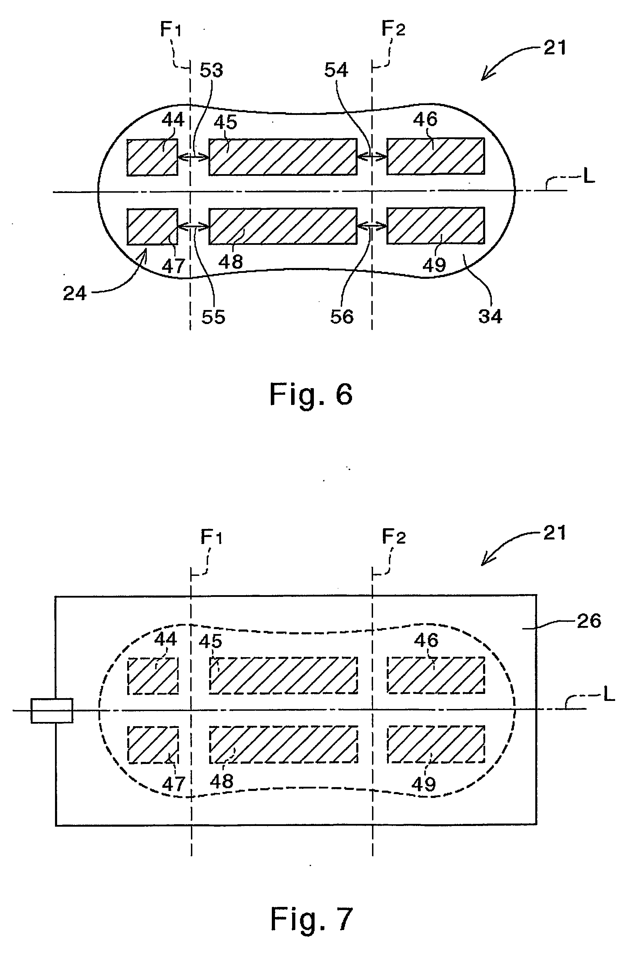

In the embodiment shown in Figs. 1 and 2, the adhesive fastener 38 can be provided in any suitable configuration known in the art (including the one depicted in Figs. 6 and 7). Preferably, , the adhesive fastener 38 is provided in the form of a pair of spaced apart longitudinally-oriented strips or zones of adhesive that are centered about the longitudinal centerline L as shown in Fig. 2.

Fig. 3 is a bottom plan view of the sanitary napkin 20 shown in Fig. 1 wherein the wrapper sheet 26 is removed for better understanding. As shown in Fig. 3, the sanitary napkin 20 further includes a removable release liner 30 for protecting the adhesive fastener 38 before use. The removable release liner 30 is releasably affixed by the adhesive fastener 38. Herein, "releasably affixed" refers to the condition of two or more components which may be attached and separated without destruction of or undue distortion to either component. The removable release liner 30 also prevents the adhesive fastener 38 from sticking to extraneous surfaces prior to use.

In a preferred embodiment, the adhesive means of the sanitary napkin 20 includes an adhesive securing means which includes a plurality of adhesive sections for securing the release liner 30 to the wrapper sheet 26. The plurality of adhesive sections are spaced apart one another by at least one non-adhesive section. In the preferred embodiment in Fig. 3, the adhesive securing means (41, 42 and 43) includes a plurality of adhesive sections 41, 42 and 43 for securing the release liner 30 to the wrapper sheet 26. The adhesive sections 41, 42 and 43 are spaced apart one another by two non-adhesive section 51 and 52. Any adhesive materials known in the art can be used for the adhesive securing means. Preferably, non-pressure sensitive adhesives are used for the adhesive securing means. A particularly preferred material for the adhesive securing means is a non-pressure sensitive adhesive supplied from Nitta Findley Co., Ltd., Osaka, Japan, under Code No. H1500U.

Herein, "non-adhesive section" refers to the portion having substantially no adhesive that contributes to an attachment of one component to another component. The non-adhesive section can take any length between two adjacent adhesive sections as long as the adhesive means (or the adhesive sections) can provide an expected function of adhesion (e.g., an attachment of the release liner 30 to the wrapper sheet 26, or an attachment of the sanitary napkin 20 to the wearer's undergarment). However, the non-adhesive section should have enough length such that no part of the adhesive sections 41, 42 and 43 comes to the edge portion(s) of the individually wrapped sanitary napkin 20 as discussed hereinafter.

In the embodiment shown in Fig. 2 wherein the sanitary napkin 20 has a longitudinal length of 205 mm and the release liner 30 has a longitudinal length of 181 mm, the adhesive sections 41, 42 and 43 have longitudinal lengths of 5 mm, 15 mm and 33 mm, respectively, and the non-adhesive sections 51 and 52 have longitudinal lengths of 48 mm and 56 mm, respectively.

Preferably, the removable release liner 30 has a relatively low light transmittance compared with the other component materials such as the wrapper sheet, backsheet or topsheet materials. In a preferred embodiment, the removable release liner 30 has a light transmittance of less than about 70%, preferably in a range of about 20-65%, and more preferably in a range of about 30-50%. These light transmittance ranges for the removable release liner 30 are preferred to provide an effective protection for the adhesive materials (i.e., the adhesive sections 41, 42 and 43) against ultraviolet light which may come through the wrapper sheet 26 when the individually wrapped sanitary napkin 20 is exposed to the ultraviolet light.

The release liner 30 can be formed by any suitable material known in the art such as paper materials, film materials and nonwoven materials. The release liner 30 is preferably provided with a release coating (preferably a silicone coating) so that the release liner 30 will release from the adhesive fastener 38 without any destruction when the wearer removes the sanitary napkin 20 from the wrapper sheet 26 for use. A preferred paper material having a silicone coated for the release liner 30 is a paper material which is supplied from Akrosil Europe B. V., Postbus, Netherlands, under Code No. BL 40g MGA Silox D3H/0 Tampopo.

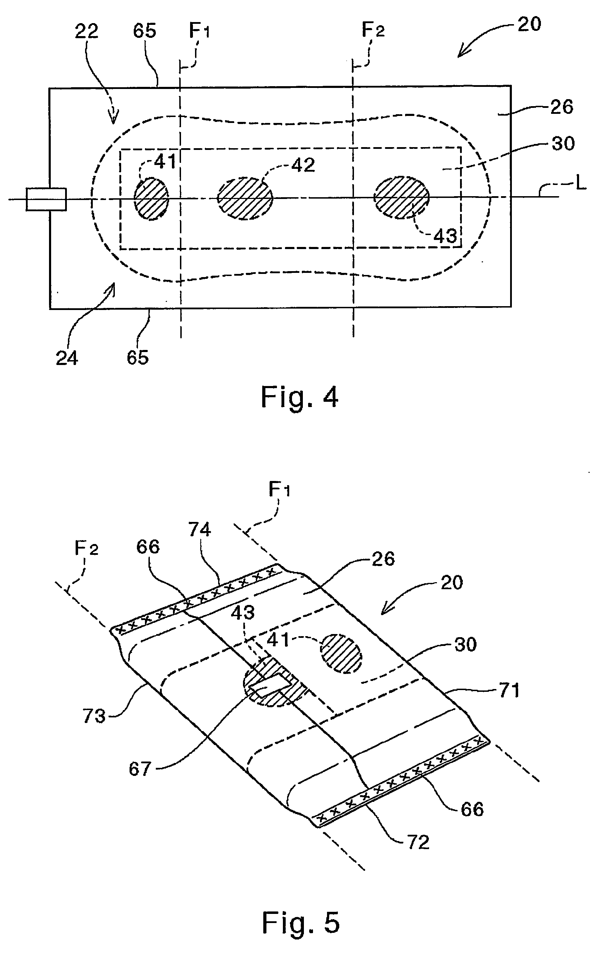

Fig. 4 is a bottom plan view of the sanitary napkin 20 of Fig. 1 wherein the wrapper sheet 26 is attached to the garment facing surface 26 of the release liner 30. The release liner 30 is secured to the body facing surface 22 of the wrapper sheet 26 by the adhesive securing means (i.e., the adhesive sections 41, 42 and 43). The wrapper sheet 26 preferably has enough area to conceal and to protect the sanitary napkin 20 in the later described folded and wrapped configurations of the sanitary napkin 20. In a preferred embodiment, the wrapper sheet 26 has enough longitudinal and lateral margins (i.e., enough longitudinal and lateral distances from the circumferential edge 29 of the main body portion of the sanitary napkin 20) which can provide a necessary sealing area for forming an individually wrapped configuration.

The wrapper sheet 26 can be made from any suitable material known in the art such as paper materials, film materials and nonwoven materials. The wrapper sheet 26 is preferably manufactured from a thin flexible material which is liquid impermeable so that the wrapper sheet 26 will be suitable for wrapping and disposing of a used sanitary napkin 20. For example, polyethylene film materials and nonwoven materials have been found to work well.

In a preferred embodiment, the wrapper sheet 26 has a light transmittance of at least about 10%, preferably in a range of about 45-100%, and more preferably in a range of about 80-100%. These light transmittance ranges for the wrapper sheet 26 are preferred to provide an effective view of the sanitary napkin 20 through the wrapper sheet 26. This is particularly important when the sanitary napkin 20 has aesthetic feature(s) (e.g., a printed graphic(s) thereon) to get consumers' strong attention.

A preferred method for measuring the light transmittance will be described in the "Test Methods" section. A particularly preferred material for the wrapper sheet 26 is a spunbonded-meltblown-spunbonded (SMS) nonwoven which is available form Mitsui Chemicals, Inc. Tokyo, Japan, under Code No. PQ-1191. The light transmittance of this wrapper sheet material is about 88%.

The sanitary napkin 20 and the wrapper sheet 26 are folded about at least one folding line that intersects the adhesive means (or the adhesive sections) at the non-adhesive section. The folding line can take any number, however, preferably one or two. For a bi-folded sanitary napkin 20, the number of the folding line is one. Alternatively, for a tri-folded sanitary napkin, the number of the folding line is two. Figs. 1-8 show preferred embodiments for such a tri-folded sanitary napkin.

The folding line can take any direction, however, preferably in the lateral direction such as those shown in Figs. 1-7. In these preferred embodiments, the sanitary napkin 20 and the wrapper sheet 26 are folded about two spaced-apart laterally oriented fold lines F1 and F2 that intersect the adhesive means (i.e., the adhesive sections 41, 42 and 43) at the non-adhesive sections 51 and 52. Herein, "spaced-apart laterally oriented fold lines" refers to longitudinally offset lines, generally parallel the lateral direction, and about which the sanitary napkin 20 including the wrapper sheet 26 is folded. Folding the sanitary napkin 20 about the spaced-apart laterally oriented fold lines F1 and F2 produces a folded arrangement defining three trisections, i.e., a central trisection 62 intermediate and bounded by two outboard trisections 61 and 63.

Fig. 5 is a perspective view of the sanitary napkin 20 which is individually wrapped by the wrapper sheet 26. The wrapper sheet 26 preferably includes means for maintaining the sanitary napkin 20 in an individually wrapped configuration thereby completing the individually wrapped structure of the sanitary napkin 20. In one preferred embodiment, each longitudinal side edge 65 of the wrapper sheet 26 is flangebly sealed to form the flanged seal 66 after the sanitary napkin 20 is folded as shown in Fig. 5. The frangible sealing of the side edges 65 of the wrapper sheet 26 can be accomplished by any suitable sealing technique known in the art. For example, the longitudinal side edges 65 may be heat sealed, glued, or ultrasonically bonded. The entire sanitary napkin 20 is thereby protected until the wrapper sheet 26 is removed by wearers' for use. Suitable methods for frangebly sealing the longitudinal side edges of the sanitary napkin 20 are disclosed in USP. Nos. 4,556,146 issued to Swanson on December 3, 1985, 5,181,610 issued to Quick et al. on January 26, 1993 and 5,462,166 issued to Minton at al. on October 31, 1995.

Preferably, the means for maintaining the sanitary napkin 20 in an individually wrapped configuration further includes an adhesive tape tab 67. The adhesive tape tab 67 is preferably joined to one end portion 68 of the wrapper sheet 26 as shown in Fig. 1. The adhesive tape tab 67 is preferably releasably attachable to the wrapper sheet 26. The adhesive tape tab 67 may be comprised of any releasably attachable and resealable material known in the art such as spots or patches of adhesive.

Preferably, the individually wrapped sanitary napkin 20 is designed such that the adhesive tape tab 67 can position above the adhesive section 43 as shown in Fig. 5. This design is preferred since the adhesive material of the adhesive section 43 can increase the physical strength of the wrapper sheet 26 at the position where the adhesive tape tab 67 is stuck, thereby preventing a possible destruction of the material when the adhesive tape tab 67 is removed to open the wrapper sheet 26.

The individually wrapped sanitary napkin 20 has four edges 71-74 (the first, second, third and fourth edges) as shown in Fig. 5. The first and third edges 71 and 73 are formed by folding the sanitary napkin 20 about the folding lines F1 and F2, respectively. The second and fourth edges 72 and 74 are formed by forming the flanged seals 66. Since the sanitary napkin 20 is folded about the fold lines F1 and F2 that intersect the adhesive sections 41, 42 and 43 of the adhesive means at the non-adhesive sections 51 and 52, the individually wrapped sanitary napkin 20 has no edge portion that contains any part of the adhesive sections 41, 42 and 43. This means that the adhesive sections 41, 42 and 43 can be protected from yellowing due to ultraviolet light if only the edge portions 71 and 73 of the individually wrapped sanitary napkin 20 is exposed to the ultraviolet light.

Fig. 6 is a bottom plan view of a sanitary napkin 21 wherein the wrapper sheet 26 is removed for better understanding, which is another preferred embodiment of the present invention. In a preferred embodiment, the adhesive means of the invention includes an adhesive fastener disposed on the garment facing surface 24 of the backsheet 34 for attaching the sanitary napkin 21 to the wearer's undergarment. The adhesive fastener includes a plurality of adhesive sections which are protected by the wrapper sheet 26. The plurality of adhesive sections are spaced apart one another by at least one non-adhesive section.

In the preferred embodiment shown in Fig. 6, the adhesive fastener includes a plurality of adhesive sections 44-49 which are spaced apart one another by four non-adhesive sections 53-56. Similarly to the embodiment shown in Fig. 2, any adhesive materials can be used for the adhesive fastener. Preferably, pressure sensitive adhesives are used for the adhesive fastener. The sanitary napkin 21 is also folded about the two folding lines F1 and F2 that intersect the adhesive means at the non-adhesive sections 44-49 as shown in Fig. 6.

In the embodiment shown in Fig. 6, the body facing surface 22 of the wrapper sheet 26 is provided with a release coating (preferably a silicone coating) so that the wrapper sheet 26 is releasably affixed to the adhesive fastener, i.e., no destruction when the wearer removes the wrapper sheet 26 for use. Preferably, such a release coating does not significantly affect (or change) the light transmittance of the wrapper sheet 26 to keep the aesthetic feature(s) which is viewed from the wrapper sheet 26.

If desired, the release coating may be provided by coating only that zone of the wrapper sheet 26 which will substantially contact the adhesive fastener (i.e., the adhesive sections 44-49). Alternatively, the entire surface of the wrapper sheet 26 may be coated. Coating the entire surface of a wrapper sheet is disclosed in USP. No. 5,181,610 issued to Quick et al. on January 26, 1993.

Fig. 7 is a bottom plan view of the sanitary napkin 21 of Fig. 6. In Fig. 7, the adhesive sections 44-49 of the adhesive fastener are protected by the wrapper sheet 26. Similarly to the embodiment shown in Fig. 1, after the sanitary napkin 21 and the wrapper sheet 26 are folded about the folding lines F1 and F2, an individually wrapped structure which is similar to the one shown in Fig. 5 will be produced. Since the sanitary napkin 21 and the wrapper sheet 26 are folded about the fold lines F1 and F2 that intersect the adhesive sections 44-49 of the adhesive means at the non-adhesive sections 53-56, the individually wrapped sanitary napkin 21 has no edge portion that contains any part of the adhesive sections 44-49. This means that the adhesive sections 44-49 can be protected from yellowing due to ultraviolet light if only the edge portions of the individually wrapped sanitary napkin 21 is exposed to the ultraviolet light.

The topsheet 32 is preferably compliant, soft feeling, and non-irritating to the wearer's skin. Further, the topsheet 32 is liquid permeable or pervious, permitting body fluids (e.g., menses and/or urine) to readily penetrate through its thickness. A suitable liquid permeable material for the topsheet may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims. When the topsheet 32 includes a nonwoven web, the web may be manufactured by a wide number of known techniques. For example, the web may be spunbonded, carded, wet-laid, melt-blown, hydroentangled, combinations of the above, or the like. The body facing surface 22 of the topsheet 32 can be made hydrophilic by treating it with a surfactant. A particularly suitable material for the topsheet 32 is a macroscopically expanded, three-dimensional formed polyethylene film is available from Tredegar Film Products, Indiana, USA under Code No. X-27121.

The backsheet 34 is impervious to body fluids and is preferably manufactured from a thin plastic film, although other flexible liquid impervious materials may also be used. Herein, "flexible" refers to materials which are compliant and will readily conform to the general shape and contours of the human body. The backsheet 34 prevents the body fluids absorbed and contained in the absorbent core 36 from wetting articles which contact the absorbent article such as bedsheets, pants, pajamas and undergarments. The backsheet 34 may thus include a woven or nonwoven material, polymeric films such as thermoplastic films of polyethylene or polypropylene, or composite materials such as a film-coated nonwoven material.

The backsheet 34 can include a single layer material, or two or more layers of materials. The backsheet 34 preferably has a thickness of from about 0.012 mm (0.5 mil) to about 0.051 mm (2.0 mils). In a preferred embodiment, the backsheet 34 is a single layer polyethylene film. Such a preferred polyethylene film is available from Swanson Plastics Corporation, Taipei, Taiwan, under Code No. JPB-168-P.

The backsheet 34 preferably has a microporous structure which can permit vapors to escape from the absorbent core (often called "breathable backsheet") while still preventing body fluids from passing through the backsheet 34. A preferred microporous polyethylene film is available from Mitsubishi Chemical Corporation, Tokyo, Japan, under Code No. NAP. The size of the garment contacting layer is dictated by the size of the absorbent core 36 and the exact absorbent article design selected.

In a preferred embodiment, the backsheet 34 has an aesthetic feature(s), for example, a graphic(s) to get consumers' attention which is printed on either the body facing surface 22 ot the garment facing surface 24. Preferred aesthetic designs include, for example, plants such as flowers (e.g., a dandelion), pretty animals such as cats, cartoon characters, seasonal things or goods such as snowmen, landscapes, and the like. The aesthetic designs can be printed by any conventional printing methods or technologies known in the art, including, but not limited to, a gravure printing, a flexo printing, an offset printing, an ink jet printing, and the like.

The absorbent core 36 is capable of receiving, absorbing or retaining body fluids discharged. The absorbent core 36 is preferably compressible, conformable, and non-irritating to the wearer's skin. The absorbent core 36 can be formed by a single layer material or a plurality layers of materials. The absorbent core may include any of a wide variety of liquid-absorbent materials known in the art, such as comminuted wood pulp, which is generally referred to as airfelt.

In a preferred embodiment, the absorbent core 36 preferably comprises a multi-bonded air laid nonwoven material. In this embodiment, this multi-bonded air laid nonwoven material comprises about 52% cellulose fibers, about 20% bi-component fibers, about 25% superabsorbent hydrogel-forming material (or absorbent gelling material) particles, and about 3% latex binder. The absorbent core 36 preferably has a basis weight of about 150 g/m2, including the particles of absorbent gelling material. A preferred multi-bonded air laid nonwoven material is obtained in roll form from BBA (China) Airlaid Co. Ltd., Tianjin, China, under the code No. B888.M.150S17.

The absorbent core 36 may be manufactured in a wide variety of sizes and shapes. The absorbent core 36 thus can take any shape in its top plan view. Preferred shapes for the absorbent core 36 include an oval, a rectangle, an hourglass, and a combination of the oval and rectangle such as the one shown in Fig. 1.

In a preferred embodiment, an acquisition layer or a secondary topsheet (not shown in Figs.) known in the art can be additionally disposed between the topsheet 32 and the absorbent core 36. The acquisition layer quickly transports discharged body fluids received by the body contacting layer 30 to other parts of the acquisition layer and the absorbent core 36, although it may temporarily hold such fluids until they can be absorbed by the absorbent core 36. In one preferred embodiment, the acquisition layer is an air laid tissue material which is available from Concert GmbH, Falkenhagen, Germany under Code No. GH082. In another preferred embodiment, the acquisition layer is a hydrophilic carded-calendered nonwoven material of polyethylene-polypropylene bi-component fiber, which is available from Kang Na Hsiung Enterprise Co., Ltd., Taipei, Taiwan, under Code No. CB018-Y.

The sanitary napkins 20 and 21 can generally have any thickness including relatively thick, intermediate thickness, relatively thin, or even very thin (or "ultra thin"). Preferred "ultra-thin" sanitary napkins which preferably have a caliper of less than about 3 mm are described in USP. Nos. 4,950,264 and 5,009,653 issued to Osborn. The embodiments of the sanitary napkins 20 and 21 shown in Figs. 1-4 are examples of an ultra-thin sanitary napkin. The sanitary napkins 20 and 21 may also be relatively flexible, so that they are comfortable for the wearer.

In preferred embodiments, the sanitary napkin 20 includes an optional pair of flaps (not shown in Figs.) which are formed by the transversely extended portion of the topsheet 32 and the backsheet 34. The flaps typically extend outward from the central region of the sanitary napkin 20.

Preferably, the flaps 44 are integral with the main body portion (i.e., the flaps comprise integral extensions of the topsheet 32 and the backsheet 34). The flaps can be in any suitable configuration. Suitable flaps are described in USP. No. 5,389,094 issued to Lavash, et al. on February 14, 1995; and USP. No. 5,558,663 issued to Weinberger, et al. on September 24, 1996.

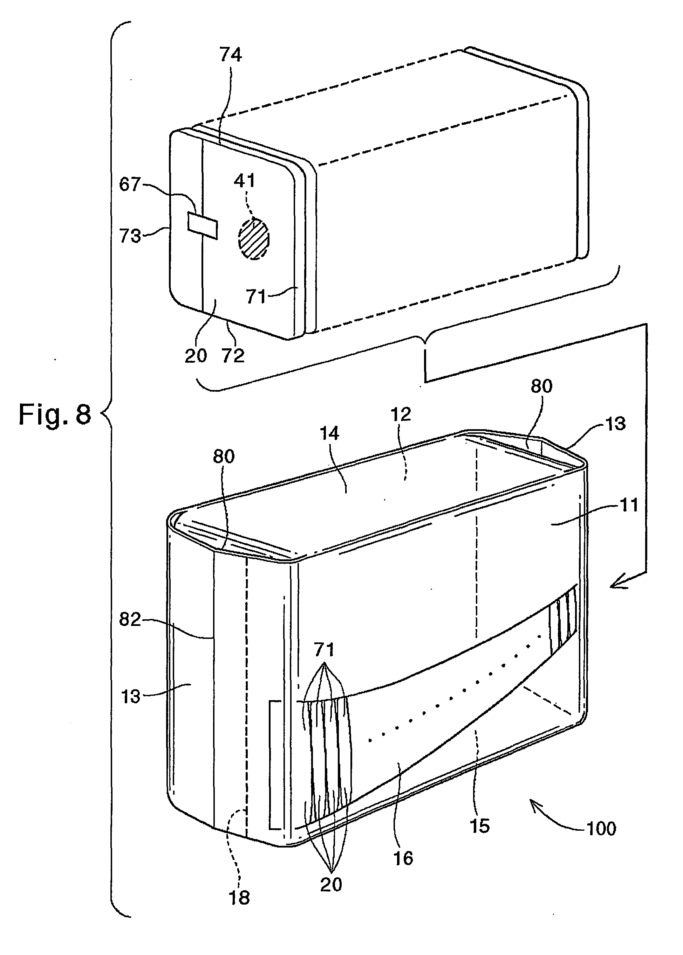

Fig. 8 is a perspective view of a disposable absorbent product containing individually wrapped sanitary napkins 20 (or 21). The individually wrapped sanitary napkins 20 are stacked and preferably compressed to form one (or more if desired) stack, and then contained in a package 100.

The package 100 can be formed by any suitable material and can take any structure known in the art. For example, the package 100 may be a cardboard box (or a carton) which is formed by a cardboard material. In the preferred embodiment shown in Fig. 8, the package 100 is a flexible bag which is formed by a thin sheet material. Such a thin sheet material may be made of paper, plastic, or any recyclable material, and may take a laminate structure comprised of two or more of the aforementioned materials. The package material may also be of non-biodegradable or non-recyclable materials, such as polymeric films (e.g., polypropylene films and polyethylene films).

The package 100 shown in Fig. 7 is a flexible bag which is preferably formed by a continuous tube of a thin film material. A preferred film material for the package 100 shown in Fig. 8 is a polyethylene film. Such a preferred polyethylene film material (transparent) is supplied from Taiwan LianBin Co., Ltd., under Code No., 95143578 and 95143581. The two side portions of the package 100 are closed by forming a side gusset structure 80 which is formed by sealing the polyethylene film along a sealing line 82. The package 100 includes a front panel 11, a rear panel 12 opposed to the front panel 11, side panels 13 which connects the front and rear panels 11 and 12, a top panel 14 which connects the front, rear, and side panels 11, 12 and 13, and a bottom panel 15 opposed to the top panel 14. Each pair of the front and rear panels 11 and 12, the side panels 13 and the top and bottom panels 14 and 15 are substantially planar as shown in Fig. 8. In this embodiment, the individually wrapped sanitary napkins 20 are staked in the direction which is perpendicular to the two side panels 13 as shown in Fig. 8, however, if preferred, they can be staked in the direction which is perpendicular to the top and bottom panels 14 and 15.

The package 100 has at least one transparent window 16. The transparent window 16 can be provided at any panels of the package 100. If desired, a plurality of transparent windows may be provided in the package 100. Alternatively, one continuous window can be formed in two (or more if possible) panels. In one embodiment, two transparent windows are provided in, for example, the front and rear panels 11 and 12. In the embodiment shown in Fig. 8, the package 100 has one transparent window 16 which is disposed in the front panel 11 (but slightly extended into the side panels 13).

The transparent window 16 should be at least translucent. Preferably the window 16 has enough transparency so that it can provide a preferred view of the individually wrapped sanitary napkins 20 in the package 100. However, the window 16 does not necessarily need to be completely transparent. The window 16 may be somewhat translucent as long as the individually wrapped sanitary napkins 20 contained in the package 100 can be seen through the window 16.

As shown in Fig. 8, each individually wrapped sanitary napkin 20 has the four edges 71-74 including the first and third edges 71 and 73 which are formed by folding the sanitary napkin 20 about the folding lines F1 and F2, respectively. Preferably, the stacked individually wrapped sanitary napkins 20 are arranged associated with the transparent window 16 such that the first edge 71 (which was formed by the folding line F1) of each sanitary napkin 20 positions towards the transparent window 16 of the package 100. This means that when the individually wrapped sanitary napkins 20 are put into the package 100, the direction of each sanitary napkin 20 is controlled with respects to the position of the transparent window 16. More specifically, as shown in the embodiment of Fig. 8, the direction of each sanitary napkin 20 is controlled so that the first edge 71 of each sanitary napkin 20 comes to the closest position against the transparent window 16 (compared with the other edges 72-74). This arrangement is advantageous since the first edge 71 of each sanitary napkin 20 has no edge portion that contains the adhesive sections 41, 42 and 43 of the adhesive means. As a result, the adhesive sections 41, 42 and 43 can be protected from yellowing caused by ultraviolet light which comes into the package 100 through the transparent window 16.

The transparent window 16 can take any shape such as a circle, a square, a rectangle, a trapezoid, an ellipse, a triangle or any other shape such as the one shown in Fig. 8. The window 16 can have any sizes or dimensions as long as the sanitary napkins 20 can be effectively seen through the window 16. The window 16 preferably has an enough dimension (e.g., the length and the width for a rectangular window) so that the contained sanitary napkins 20 can be seen through the window 16. Preferably, the window 16 has an enough size so that at least 30%, more preferably about from 60% to about 100% of the contained sanitary napkins 20 can be seen through the window 16. The window 16 enables users to consume the plurality of types of sanitary napkins 20 equally. The window 16 also enables consumers (or purchasers) to understand the plurality of types of sanitary napkins 20 are contained within the package 100 at the point of purchase.

The transparent window 16 can be formed by any means known in the art. Preferably, the window 16 is formed by differentiating the translucency or the transparency at the window 16 from the area surrounding the window 16. When a transparent polyethylene film is used for the package 100, the translucency or the transparency of the window 16 can be controlled by changing an amount of paint(s) or ink(s) to be used for the package 100. Alternatively, the translucency or the transparency of the window 16 can be controlled by using different paints between the area within the window 16 and the area surrounding the window 16. In the embodiment shown in Fig. 8, the window 16 can be formed by applying no paint or ink in the area of the window 16 so that it has a high transparency, while applying a paint(s) which makes the surrounding area of the window 16 (including the area which is needed to form the sealing line 82 in the side panels 13) non-transparent. Preferably, no portions other than the transparent window(s) 16 should be non- transparent or at least translucent.

In the embodiment wherein the package 100 is formed by a cardboard material (not shown in Figs.), the window 16 is formed by removing at least one part of the cardboard material by cutting: Preferably, the cut portion is covered by a separate sheet material (e.g., a translucent or transparent film material) which is attached to the inside surface of the package wall at the window portion so that the contained sanitary napkins 20 can be effectively protected from the outside.

Preferably, an opening device 18 is provided within one of the side panel 13. The opening device 18 preferably has an enough size (e.g., the length) so that the sanitary napkins 20 can be picked up easily by the user. The opening device 18 can take any structure, shape and dimension known in the art. The opening device 18 preferably includes a line of weakness which extends within the side panel 13. In the embodiment shown in Fig. 8, the line of weakness includes a line of perforation formed in the side panel 13. If desired, the line of weakness may extend into the other panels from the side panel 13.

TEST METHODS

This section describes one preferred method for determining the light transmittance of a sheet material. It should be noted that other method known in the art for determining the light transmittance can be applicable.

A light transmittance analyzer is preferably used for determining the light transmittance of a sample sheet material. A preferred light transmittance analyzer is available from Nomura Shoji Co., Ltd., Tokyo, Japan, under Trade Name "Formation Tester" and Code No. FMT-2000. This light transmittance analyzer includes a light source which has a Halogen lamp (12V, 75W), a sample holder table having an open window, a CCD camera (256 x 243 pixels), and a computer. The light source is placed away from one side of the sample holder table, while the CCD camera is placed away from the other side of the sample holder table. The distance between the lens of the CCD camera and the sample holder table is about 415 mm. The open area of the window is adjusted so that its effective open area has a square of 40 x 45 mm.

In the measurement, the Halogen lamp turns on. When no sample sheet material is held by the sample holder table, the reference light volume (Vr) is measured by the CCD camera and recorded by the computer. A sample sheet material is held by the sample holder table so that it receives the light irradiated from the Halogen lamp in the effective open area. The light passes through the sample sheet material and reaches the CCD camera. The sample light volume (Vs) is then measured by each pixel of the CCD camera and recorded by the computer. This process is repeated for one sample sheet material at least three times and the average values of the light volumes (Vrav and Vsav) are calculated and recorded by the computer. The computer then calculates the light transmittance (LT) by the following formula:

$$LT = (Vsav \; / \; Vrav) \; x \; 100 \; (\%) \qquad\qquad \text{--- (1)}$$

All documents cited in the Detailed Description of the Invention are, are, in relevant part, incorporated herein by reference; the citation of any document is not to be construed as an admission that it is prior art with respect to the present invention.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

**Claims**

1.    A disposable absorbent product, comprising:

a package (100) having a transparent window (16); and
a plurality of individually wrapped disposable absorbent articles (20) which are stacked and contained in the package (100);
each absorbent article having a longitudinal centerline (L) and a transverse centerline (T), and including a liquid impermeable backsheet (34) having a garment facing surface, an adhesive means disposed in the longitudinal direction, and a wrapper sheet (26) for individually wrapping the absorbent article, the adhesive means being disposed between the garment facing surface of the backsheet (34) and the wrapper sheet (26);
the adhesive means including a plurality of adhesive sections (41-49) which are spaced apart one another by at least one non-adhesive section (51-56), the wrapper sheet (26) covering the adhesive means and the garment facing surface of the backsheet, each absorbent article being folded about at least one folding line (F1) that intersects the adhesive means at the non-adhesive section (51-56) <u>such that the folding line (F1) contains no adhesive section (41-49)</u>;
wherein the stacked plurality of the individually wrapped absorbent articles (20) are arranged associated with the transparent window (16) such that the folding line (F1) of each absorbent article (20) positions towards the transparent window(16) of the package (100).

2.    The disposable absorbent product of the claim 1,
each absorbent article including (a) an adhesive fastener (38) disposed on the garment facing surface of the backsheet for attaching the absorbent article to the wearer's undergarment, and (b) a release liner (30) for protecting the adhesive fastener (38),
the adhesive means including an adhesive securing means (41, 42, 43) which includes the plurality of adhesive sections (41, 42, 43) for securing the release liner (30) to the wrapper sheet (26).

3.    The disposable absorbent product of the claim 1,
the adhesive means including an adhesive fastener (38) including the plurality of adhesive sections (44-49) which are disposed on the garment facing surface of the backsheet (34) for attaching the absorbent article to the wearer's undergarment.

4. The disposable absorbent product of the claim 1, wherein the wrapper sheet (26) includes a nonwoven material.

5. The disposable absorbent product of the claim 1,
the package including a front panel (11), a rear panel (12) opposed to the front panel (11), two side panels (13) which connects the front and rear panels, a top panel (14), and a bottom panel (15) opposed to the top panel, the top and bottom panels connecting the front, rear, and side panels, the transparent window (16) being formed on the front panel (11), and
the plurality of the individually wrapped absorbent articles (20) being staked in a direction which is perpendicular to the two side panels (13)or the top and bottom panels (14,15).

6. The disposable absorbent product of the claim 1,
the at least one folding line including first and second folding lines (F1, F2),
each absorbent article (20) being folded about the first and second folding lines (F1, F2), and the first folding line (F1) intersecting the adhesive means at the non-adhesive section (51),
wherein the stacked plurality of the individually wrapped absorbent articles are arranged associated with the transparent window (16) such that the first folding line (F1) of each absorbent article positions towards the transparent window (16) of the package (100).

7. The disposable absorbent product of the claim 1, wherein the disposable absorbent article is a sanitary napkin or a pantyliner.

8. The disposable absorbent product of the claim 1, wherein the plurality of adhesive sections (41-49) of each absorbent article are not seen through the wrapper sheet and the transparent window.

9. A disposable absorbent product, comprising:

a package (100) having a transparent window (16); and
a plurality of individually wrapped disposable absorbent articles (20) which are stacked and contained in the package (100);
each absorbent article having a longitudinal centerline (L) and a transverse centerline (T), and including a liquid impermeable backsheet (34) having a garment facing surface, an adhesive fastener disposed on the garment facing surface of the backsheet for attaching the absorbent article to the wearer's undergarment, a release liner for protecting the adhesive fastener, a wrapper sheet for wrapping the absorbent article, an adhesive means including an adhesive securing means which includes a plurality of adhesive sections (41,42,43) for securing the release liner to the wrapper sheet (26);
the plurality of adhesive sections (41,42,43) are spaced apart one another by at least one non-adhesive section (51,52), each absorbent article (20) being folded about at least one folding line (F1) that intersects the adhesive means at the non-adhesive section (51,52) such that the folding line (F1) contains no adhesive section (41,42,43);
wherein the stacked plurality of the individually wrapped absorbent articles (20) are arranged associated with the transparent window (16) such that the folding line (F1) of each absorbent article positions towards the transparent window (16) of the package.

10. A disposable absorbent product, comprising:

a package (100) having a transparent window (16); and
a plurality of individually wrapped disposable absorbent articles (20) which are stacked and contained in the package (100);
each absorbent article (20) having a longitudinal centerline (L) and a transverse centerline (T), and including a liquid impermeable backsheet (34) having a garment facing surface, an adhesive fastener (38) disposed on the garment facing surface of the backsheet (34) for attaching the absorbent article to the wearer's undergarment, the adhesive fastener (38) including a plurality of adhesive sections (44-49), and a wrapper sheet (26) for protecting the adhesive fastener (38) and wrapping the absorbent article;
the plurality of adhesive sections (44-49)are spaced apart one another by at least one non-adhesive section (53-56), each absorbent article being folded about at least one folding line (F1) that intersects the adhesive means at the non-adhesive section <u>such that the folding line (F1) contains no adhesive section (44-49);</u>
wherein the stacked plurality of the individually wrapped absorbent articles are arranged associated with the transparent window such that the folding line (F1) of each absorbent article positions towards the transparent window of the package.

## Patentansprüche

1. Einweg-Absorptionsprodukt, das Folgendes umfasst:

   eine Verpackung (100) mit einem transparenten Fenster (16); und
   mehrere einzeln verpackte Einweg-Absorptionsartikel (20), die in der Verpackung (100) gestapelt und darin enthalten sind;
   wobei jeder Absorptionsartikel eine Längsachse (L) und eine Querachse (T) aufweist und eine flüssigkeitsundurchlässige Unterschicht (34) mit einer bekleidungsseitigen Oberfläche, ein in Längsrichtung angeordnetes Haftmittel und eine Schutzhüllenfolie (26) zum einzelnen Verpacken des Absorptionsartikels enthält, wobei das Haftmittel zwischen der bekleidungsseitigen Oberfläche der Unterschicht (34) und der Schutzhüllenfolie (26) angeordnet ist;
   wobei das Haftmittel mehrere Haftmittelabschnitte (41-49) umfasst, die voneinander durch mindestens einen nichtklebenden Abschnitt (51-56) getrennt sind, wobei die Schutzhüllenfolie (26) das Haftmittel und die bekleidungsseitige Oberfläche der Unterschicht abdeckt, wobei jeder Absorptionsartikel um mindestens eine Faltlinie (F1) gefaltet wird, die das Haftmittel an dem nichtklebenden Abschnitt (51-56) <u>solcherart überschneidet, dass die Faltlinie (F1) einen haftmittelfreien Abschnitt (41-49) enthält;</u>
   wobei die mehreren gestapelten, einzeln verpackten Absorptionsartikel (20) bezüglich des transparenten Fensters (16) so angeordnet sind, dass die Faltlinie (F1) eines jeden Absorptionsartikels (20) in Richtung des transparenten Fensters (16) der Verpackung (100) positioniert ist.

2. Einweg-Absorptionsprodukt nach Anspruch 1,
   wobei jeder Absorptionsartikel (a) ein selbstklebendes Befestigungsmittel (38), das an der bekleidungsseitigen Oberfläche der Unterschicht zum Befestigen des Absorptionsartikels an der Unterwäsche des Trägers angebracht ist, und (b) einen Release-Liner (30) zum Schutz des selbstklebenden Befestigungsmittels (38) enthält,
   wobei das Haftmittel ein selbst klebendes Befestigungsmittel (41, 42, 43) enthält, das mehrere Haftmittelabschnitte (41, 42, 43) aufweist, um den Release-Liner (30) an der Schutzhüllenfolie (26) zu befestigen.

3. Einweg-Absorptionsprodukt nach Anspruch 1,
   wobei das Haftmittel ein selbstklebendes Befestigungsmittel (38) enthält, das mehrere Haftmittelabschnitte (44-49) aufweist, die an der bekleidungsseitigen Oberfläche der Unterschicht (34) befestigt sind, um den Absorptionsartikel an der Unterwäsche des Trägers zu befestigen.

4. Einweg-Absorptionsprodukt nach Anspruch 1, wobei die Schutzhüllenfolie (26) ein Vliesmaterial enthält.

5. Einweg-Absorptionsprodukt nach Anspruch 1,
   wobei die Verpackung eine Vorderseite (11), eine der Vorderseite (11) entgegengesetzte Rückseite (12), zwei die Vorder- und die Rückseite miteinander verbindende Seitenteile (13), eine Oberseite (14) und eine der Oberseite entgegengesetzte Unterseite (15) aufweist, wobei die Ober- und die Unterseite die Vorder-, Rück- und Seitenteile miteinander verbinden,
   wobei das transparente Fenster (16) auf der Vorderseite (11) gebildet ist und
   die mehreren, einzeln verpackten Absorptionsartikel (20) in eine Richtung gestapelt werden, die senkrecht zu den beiden Seitenteilen (13) bzw. der Ober- und Unterseite (14, 15) verläuft.

6. Einweg-Absorptionsprodukt nach Anspruch 1,
   wobei die mindestens eine Faltlinie eine erste und eine zweite Faltlinie (F1, F2) enthält,
   wobei jeder Absorptionsartikel (20) um die erste und zweite Faltlinie (F1, F2) gefaltet wird und die erste Faltlinie (F1) das Haftmittel an dem haftmittelfreien Abschnitt (51) überschneidet,
   wobei die mehreren gestapelten, einzeln verpackten Absorptionsartikel bezüglich des transparenten Fensters (16) so angeordnet sind, dass die Faltlinie (F1) eines jeden Absorptionsartikels in Richtung des transparenten Fensters (16) der Verpackung (100) positioniert ist.

7. Einweg-Absorptionsprodukt nach Anspruch 1, wobei der Einweg-Absorptionsartikel eine Damenbinde oder eine Slipeinlage ist.

8. Einweg-Absorptionsprodukt nach Anspruch 1, wobei die mehreren Haftmittelabschnitte (41-49) eines jeden Absorptionsartikels durch die Schutzhüllenfolie und das transparente Fenster hindurch nicht sichtbar sind.

9. Einweg-Absorptionsprodukt, das Folgendes umfasst:

eine Verpackung (100) mit einem transparenten Fenster (16); und
mehrere einzeln verpackte Einweg-Absorptionsartikel (20), die in der Verpackung (100) gestapelt und enthalten sind;
wobei jeder Absorptionsartikel eine Längsachse (L) und eine Querachse (T) aufweist und eine flüssigkeitsundurchlässige Unterschicht (34) mit einer bekleidungsseitigen Oberfläche enthält sowie ein selbstklebendes Befestigungsmittel (38), das an der bekleidungsseitigen Oberfläche der Unterschicht zum Befestigen des Absorptionsartikels an der Unterwäsche des Trägers angebracht ist, eine Schutzhüllenfolie zum Verpacken des Absorptionsartikels, ein Haftmittel, das ein selbstklebendes Befestigungsmittel (41, 42, 43) umfasst, das mehrere Haftmittelabschnitte (41, 42, 43) aufweist, um den Release-Liner an der Schutzhüllenfolie (26) zu befestigen;
wobei die mehreren Haftmittelabschnitte (41, 42, 43) voneinander durch mindestens einen nichtklebenden Abschnitt (51, 52) getrennt sind, wobei jeder Absorptionsartikel (20) um mindestens eine Faltlinie (F1) gefaltet wird, die das Haftmittel an dem nichtklebenden Abschnitt (51, 52) solcherart überschneidet, dass die Faltlinie (F1) keinen haftmittelfreien Abschnitt (41, 42, 43) enthält;
wobei die mehreren gestapelten, einzeln verpackten Absorptionsartikel (20) bezüglich des transparenten Fensters (16) so angeordnet sind, dass die Faltlinie (F1) eines jeden Absorptionsartikels in Richtung des transparenten Fensters (16) der Verpackung positioniert ist.

10. Einweg-Absorptionsprodukt, das Folgendes umfasst:

eine Verpackung (100) mit einem transparenten Fenster (16); und
mehrere einzeln verpackte Einweg-Absorptionsartikel (20), die in der Verpackung (100) gestapelt und enthalten sind;
wobei jeder Absorptionsartikel (20) eine Längsachse (L) und eine Querachse (T) aufweist und eine flüssigkeitsundurchlässige Unterschicht (34) mit einer bekleidungsseitigen Oberfläche, ein selbstklebendes Befestigungsmittel (38), das an der bekleidungsseitigen Oberfläche der Unterschicht (34) zum Befestigen des Absorptionsartikels an der Unterwäsche des Trägers angebracht ist, wobei das selbstklebende Befestigungsmittel (38) mehrere Haftmittelabschnitte (44-49) und eine Schutzhüllenfolie (26) zum Schutz des selbstklebenden Befestigungsmittels (38) und zum Verpacken des Absorptionsartikels enthält;
wobei die mehreren Haftmittelabschnitte (44-49) voneinander durch mindestens einen nichtklebenden Abschnitt (53-56) getrennt sind, wobei jeder Absorptionsartikel um mindestens eine Faltlinie (F1) gefaltet wird, die das Haftmittel an dem nichtklebenden Abschnitt solcherart überschneidet, dass die Faltlinie (F1) keinen haftmittelfreien Abschnitt (44-49) enthält;
wobei die mehreren gestapelten, einzeln verpackten Absorptionsartikel bezüglich des transparenten Fensters so angeordnet sind, dass die Faltlinie (F1) eines jeden Absorptionsartikels in Richtung des transparenten Fensters der Verpackung positioniert ist.

**Revendications**

1. Produit absorbant jetable, comprenant :

un conditionnement (100) ayant une fenêtre transparente (16) ; et
une pluralité d'articles absorbants jetables enveloppés individuellement (20) qui sont empilés et contenus dans le conditionnement (100) ;
chaque article absorbant ayant une ligne médiane longitudinale (L) et une ligne médiane transversale (T), et incluant une feuille de fond imperméable aux liquides (34) ayant une surface faisant face au vêtement, un moyen adhésif disposé dans la direction longitudinale, et une feuille d'enveloppement (26) pour envelopper individuellement l'article absorbant, le moyen adhésif étant disposé entre la surface faisant face au vêtement de la feuille de fond (34) et la feuille d'enveloppement (26) ;
le moyen adhésif incluant une pluralité de sections adhésives (41-49) qui sont espacées l'une de l'autre par au moins une section non adhésive (51-56), la feuille d'enveloppement (26) couvrant le moyen adhésif et la surface faisant face au vêtement de la feuille de fond, chaque article absorbant étant plié autour d'au moins une ligne de pliage (F1) qui coupe le moyen adhésif au niveau de la section non adhésive (51-56) de telle sorte que la ligne de pliage (F1) ne contient aucune section adhésive (41-49) ;

dans lequel la pluralité empilée des articles absorbants enveloppés individuellement (20) est arrangée associée à la fenêtre transparente (16) de telle sorte que la ligne de pliage (F1) de chaque article absorbant (20) se positionne en direction de la fenêtre transparente (16) du conditionnement (100).

**2.** Produit absorbant jetable selon la revendication 1,

chaque article absorbant incluant (a) un fermoir adhésif (38) disposé sur la surface faisant face au vêtement de la feuille de fond pour fixer l'article absorbant au sous-vêtement du porteur, et (b) une protection détachable (30) pour protéger le fermoir adhésif (38),

le moyen adhésif incluant un moyen de fixation adhésif (41, 42, 43) qui inclut la pluralité de sections adhésives (41, 42, 43) pour fixer la protection détachable (30) à la feuille d'enveloppement (26).

**3.** Produit absorbant jetable selon la revendication 1,

le moyen adhésif incluant un fermoir adhésif (38) incluant la pluralité de sections adhésives (44-49) qui sont disposées sur la surface faisant face au vêtement de la feuille de fond (34) pour fixer l'article absorbant au sous-vêtement du porteur.

**4.** Produit absorbant jetable selon la revendication 1, dans lequel la feuille d'enveloppement (26) inclut un matériau non tissé.

**5.** Produit absorbant jetable selon la revendication 1,

le conditionnement incluant un panneau avant (11), un panneau arrière (12) opposé au panneau avant (11), deux panneaux latéraux (13) qui relient les panneaux avant et arrière, un panneau supérieur (14), et un panneau inférieur (15) opposé au panneau supérieur, les panneaux supérieur et inférieur reliant les panneaux avant, arrière, et latéraux, la fenêtre transparente (16) étant formée sur le panneau avant (11), et

la pluralité des articles absorbants enveloppés individuellement (20) étant empilée dans une direction qui est perpendiculaire aux deux panneaux latéraux (13) ou aux panneaux supérieur et inférieur (14, 15).

**6.** Produit absorbant jetable selon la revendication 1,

l'au moins une ligne de pliage incluant des première et deuxième lignes de pliage (F1, F2),

chaque article absorbant (20) étant plié autour des première et deuxième lignes de pliage (F1, F2), et la première ligne de pliage (F1) croisant le moyen adhésif au niveau de la section non adhésive (51),

dans lequel la pluralité empilée des articles absorbants enveloppés individuellement est arrangée associée à la fenêtre transparente (16) de telle sorte que la première ligne de pliage (F1) de chaque article absorbant se positionne en direction de la fenêtre transparente (16) du conditionnement (100).

**7.** Produit absorbant jetable selon la revendication 1, où l'article absorbant jetable est une serviette hygiénique ou un protège-slip.

**8.** Produit absorbant jetable selon la revendication 1, dans lequel la pluralité de sections adhésives (41-49) de chaque article absorbant n'est pas visible à travers la feuille d'enveloppement et la fenêtre transparente.

**9.** Produit absorbant jetable, comprenant :

un conditionnement (100) ayant une fenêtre transparente (16) ; et

une pluralité d'articles absorbants jetables enveloppés individuellement (20) qui sont empilés et contenus dans le conditionnement (100) ;

chaque article absorbant ayant une ligne médiane longitudinale (L) et une ligne médiane transversale (T), et incluant une feuille de fond imperméable aux liquides (34) ayant une surface faisant face au vêtement, un fermoir adhésif disposé sur la surface faisant face au vêtement de la feuille de fond pour fixer l'article absorbant au sous-vêtement du porteur, une protection détachable pour protéger le fermoir adhésif, une feuille d'enveloppement pour envelopper l'article absorbant, un moyen adhésif incluant un moyen de fixation adhésif qui inclut une pluralité de sections adhésives (41, 42, 43) pour fixer la protection détachable à la feuille d'enveloppement (26) ;

la pluralité de sections adhésives (41, 42, 43) est espacée l'une de l'autre par au moins une section non adhésive (51, 52), chaque article absorbant (20) étant plié autour d'au moins une ligne de pliage (F1) qui coupe le moyen adhésif au niveau de la section non adhésive (51, 52) de telle sorte que la ligne de pliage (F1) ne contient aucune section adhésive (41, 42, 43) ;

dans lequel la pluralité empilée des articles absorbants enveloppés individuellement (20) est arrangée associée à la fenêtre transparente (16) de telle sorte que la ligne de pliage (F1) de chaque article absorbant se positionne en direction de la fenêtre transparente (16) du conditionnement.

10. Produit absorbant jetable, comprenant :

un conditionnement (100) ayant une fenêtre transparente (16) ; et

une pluralité d'articles absorbants jetables enveloppés individuellement (20) qui sont empilés et contenus dans le conditionnement (100) ;

chaque article absorbant (20) ayant une ligne médiane longitudinale (L) et une ligne médiane transversale (T), et incluant une feuille de fond imperméable aux liquides (34) ayant une surface faisant face au vêtement, un fermoir adhésif (38) disposé sur la surface faisant face au vêtement de la feuille de fond (34) pour fixer l'article absorbant au sous-vêtement du porteur, le fermoir adhésif (38) incluant une pluralité de sections adhésives (44-49), et une feuille d'enveloppement (26) pour protéger le fermoir adhésif (38) et envelopper l'article absorbant ;

la pluralité de sections adhésives (44-49) est espacée l'une de l'autre par au moins une section non adhésive (53-56), chaque article absorbant étant plié autour d'au moins une ligne de pliage (F1) qui coupe le moyen adhésif au niveau de la section non adhésive de telle sorte que la ligne de pliage (F1) ne contient aucune section adhésive (44-49):

dans lequel la pluralité empilée des articles absorbants enveloppés individuellement est arrangée associée à la fenêtre transparente de telle sorte que la ligne de pliage (F1) de chaque article absorbant se positionne en direction de la fenêtre transparente du conditionnement.

Fig. 1

Fig. 2

Fig. 3

Fig. 4

Fig. 5

Fig. 6

Fig. 7

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6074376 A, Mills
- US 5569228 A, Byrd
- US 5413568 A, Roach
- WO 02096331 A
- US 4556146 A, Swanson
- US 5181610 A, Quick
- US 5462166 A, Minton
- US 4950264 A
- US 5009653 A, Osborn
- US 5389094 A, Lavash
- US 5558663 A, Weinberger